# EUROPEAN PATENT APPLICATION

(11) **EP 3 847 963 A1**
(43) Date of publication of application: **14.07.2021**
(21) Application number: 20150887.6
(22) Date of filing: 09.01.2020
(51) Int. Cl.: A61B 5/1455, A61B 5/053

(54) **PROBE AND MEASUREMENT SYSTEM**

(71) Applicant: CDIA Asset Holding AB, 117 29 Stockholm (SE)
(72) Inventor: GELADI, Paul, 906 54 Umea (SE); GRAHN, Hans, 146 52 Tullinge (SE)
(74) Representative: AWA Sweden AB

(57) **Abstract**

A probe (200) for combined impedance and light absorbance measurement on skin (202) comprises a two-dimensional array (206) of skin-facing sub-probes (208), each skin-facing sub-probe (208) comprising an optical fiber end (210, 211) coated, around its circumference, with an electrode (212). A measurement system (100) is configured for simultaneous impedance and light measurement on skin (202), and comprises the probe (200), an impedance measurement device (118) configured for pairwise impedance measurement across a plurality of pairs of electrodes (212) of the skin-facing sub-probes (208); and a light measurement device (112) configured for the light absorbance measurement through a plurality of optical fiber ends (210, 211) of the skin-facing sub-probes.

## Description

### Field of technology

The present disclosure relates to a probe for combined impedance and light absorbance measurement and a measurement system configured for the same.

### Background

Devices, probes and systems for combined impedance and light absorbance measurement on skin, in particular human skin, are known in the prior art. They find use, for example, in diagnostics of skin cancer, such as malign melanoma, or neuropathy. Other skin anomalies for which such probes are useful include erythema, e.g., from diabetes patients, burn wounds, healing processes of surgical wounds and scars, acne, psoriasis, other wounds, etc.

Especially, near infra-red (NIR) spectral information has been used in combination with impedance measurements for such diagnostics.

Document WO02/056766 A1 discloses such a probe.

### Definitions

Throughout this disclosure, the term "light" should be understood as referring to any electromagnetic radiation which an optical fiber is capable of carrying, including infra-red, near infra-red (NIR), visible, and ultra-violet radiation.

"Near infra-red" (NIR) should be understood as the approximate electromagnetic wavelength interval 800 nm - 2500 nm.

"Optical fiber" and "optical fiber end" may, in the following, be understood, unless otherwise noted, to refer to either single optical fibers or bundles of two or more optical fibers.

### Summary

It is an object of the present disclosure to provide an improved probe and measurement system for combined impedance and light absorbance measurement on skin.

To this end, there is provided a probe for combined impedance and light absorbance measurement on skin, comprising a two-dimensional array of skin-facing sub-probes, each said skin-facing sub-probe comprising an optical fiber end coated, around its circumference, with an electrode.

Such a probe, with a fiberoptic-electrode sub-probe combination, with each sub-probe comprising an optical fiber end coated with conductive material, allows for an optical absorption measurement at each fiber, and, moreover, pairwise skin impedance measurements in many combinations between the electrodes coating each fiber. Hereby, a multi-mode, multi-location probe is provided, i.e., a probe providing combined and collocated multi-modal information, i.e., electrical and optical information, at a plurality of locations. For example, several measurements at different locations on a skin spot may be made at once, together with measurements at adjacent healthy skin for comparison and reference.

Thus, by using the probe, in one skin measurement step, local multi-modal and potential multi-spectral data may be provided for a plurality of skin locations covering a relatively large area of skin.

Moreover, by combining each optical fiber with an electrode, a particularly compact probe may be provided, as separate electrodes and optical sub-probes are not required, allowing for skin measurements to be performed at high spatial resolution, in turn allowing for contours of absorbance and impedance, or parameters calculated from multivariate data derived therefrom, to be reconstructed at high accuracy. Hereby, size variations in skin spots may outlined in more detail.

The electrode material may be adapted according to needs. In general, the electrode be made from any electrically-conductive material, for example from metal, from conducting carbon, or from conducting polymer.

Around its circumference, the optical fiber end may be at least partially, or completely, coated by the electrode. The fiber material may be adapted to the spectroscopy technique used. For example, an optical fiber may be a quartz fiber or each of the fibers may be made from quartz fiber.

The sub-probes of the two-dimensional array may be arranged in any suitable spatial pattern, for example a square, rectangular, circular, elliptic, or hexagonal pattern.

A fiber end, not counting the electrode, may have a maximum transversal extent, such as a diameter in the case of a cylindrical fiber end, in the interval, 0.25 mm - 4.0 mm, and preferably 0.5 mm - 2.0 mm.

The electrode may have a maximum transversal thickness in the interval, 0.03 mm - 0.5 mm, and preferably 0.06 mm - 0.25 mm.

Further, there is provided a measurement system configured for simultaneous impedance and light measurement on skin, comprising the probe according to the above; an impedance measurement device configured for pairwise impedance measurement across a plurality of pairs of electrodes of the skin-facing sub-probes; and a light measurement device configured for the light absorbance measurement through a plurality of optical fiber ends of the skin-facing sub-probes.

The measurement system may generally be associated with the same features and advantages as the probe according to the above.

The system may further comprise a plurality of microswitches configured to switchably connect pairs of electrodes of the plurality of electrodes to the impedance measurement device for the pairwise impedance measurement. The use of microswitches makes practical pairwise impedance measurements in many combinations between the electrodes coating each fiber.

The system may be configured for the impedance measurement at a plurality of frequencies. This may provide multivariate-data useful for skin analysis.

The system may further comprise an array of optical switches configured to switchably connect a fiber connected to a fiber end of the plurality of optical fiber ends to the light measurement device for the light absorbance measurement. This allows for measurements using the all sub-probe fiber end using a single light measurement device.

The light measurement device may be configured for near infra-red (NIR) light absorbance measurement.

The light measurement device may be configured for the light absorbance measurement at a plurality of wavelengths. This may provide multivariate-data useful for skin analysis.

The light measurement device may further comprise a processor configured to interpolate and/or extrapolate a map of skin properties based on the combined impedance and light measurement.

The probe as described above, or the system as described above, may be used in combined impedance and light absorbance measurement on skin.

### Brief description of the drawings

The above, as well as additional objects, features and advantages of the present inventive concept, will be better understood through the following illustrative and non-limiting detailed description of preferred embodiments, with reference to the appended drawings, where the same reference numerals will be used for similar elements, wherein:
Fig. 1 schematically shows a measurement system configured for simultaneous impedance and light measurement on skin, including a probe configured for the same,
Fig. 2 shows a front surface of the probe,
Figs 3a and 3b show, respectively, in a cross-sectional view and in a front view, a sub-probe of the probe according to a first configuration,
Figs 4a and 4b show, respectively, in a cross-sectional view and in a front view, a sub-probe of the probe, according to a second configuration, and
Fig. 5 shows a typical measurement sequence.

### Detailed description

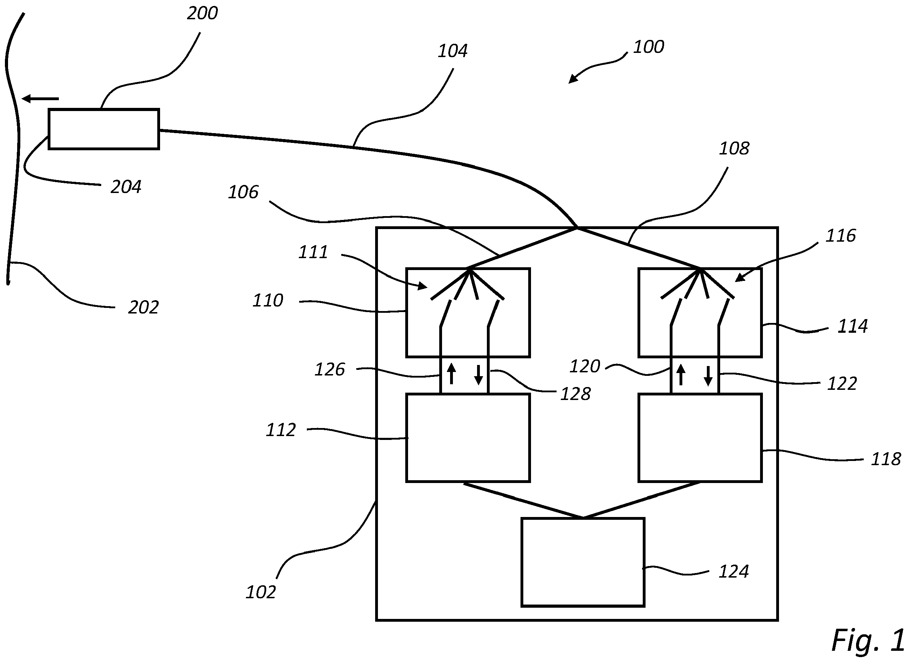

Fig. 1 schematically shows a measurement system 100 configured for simultaneous impedance and light measurement on human or animal skin 202. The measurement system comprises a probe 200 and a main unit 102, connected by a combined electric and optical cable 104. During measurement, the probe may be pushed against an area of interest of the skin 202, as indicated by an arrow. Typically, the probe may be around 20 cm long and a few centimeters in transversal extent, such as a diameter.

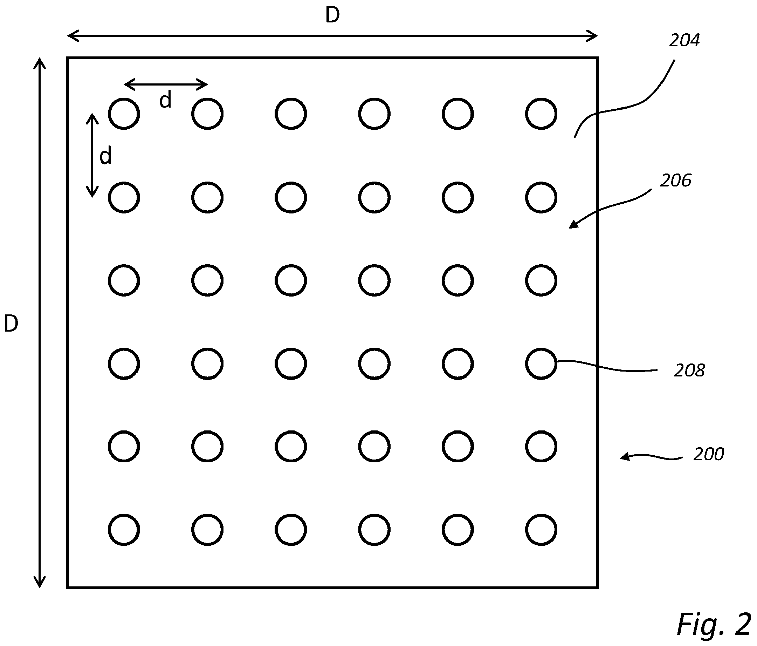

Distributed over a front surface 204, the probe 200 comprises a two-dimensional array of sub-probes 208 (cf. Figs 2, 3a, 3b, 4a, 4b).

Fig. 2 shows the front surface 204 of the probe 200. The front surface 204 comprises a two-dimensional array 206 of (schematically drawn) skin-facing sub-probes 208. As shown, the sub-probes 208 in the array 206 may be disposed in a repeating pattern, in particular a square pattern at a center spacing d. However rectangular, circular, elliptic, or hexagonal patterns, which may be repeating patterns, are equally possible.

The sub-probe spacing *d* may for example be in the interval, 1 mm - 5 mm.

The front surface 204 may have a maximum transversal extent D, as shown a square side, in the range 3 mm - 20 mm, and preferably 5 mm - 12 mm. A typical extent *D* may be about 10 mm. These are sizes well adapted to typical skin spots sizes to be examined, typically allowing for a spot of interest and adjacent healthy skin to be covered by the front surface 204, allowing examination of both, for comparison, in a single step.

As shown, the front surface 204 may have 6 x 6 = 36 sub-probes, however, larger or smaller arrays are equally possible, for example a 3 x 3 array. Array size and sub-probe spacing *d* may be decided based on achievable resolution in relation to available data acquisition hardware and/or computing power at a given cost.

Each sub-probe 208 has means for combined impedance and light absorbance measurement on skin 202, as will be detailed below.

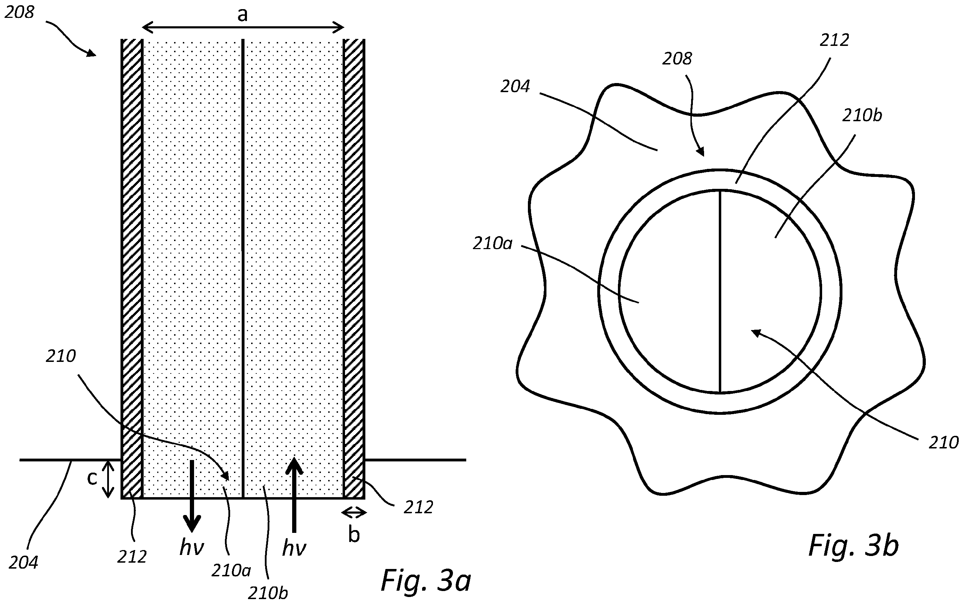

Figs 3a and 3b show, in a cross-sectional view (Fig. 3a) and a front view (Fig. 3b), a skin-facing sub-probe 208 of the array of skin-facing sub-probes 206 (cf. Fig. 2) as located at the front surface 204 of the probe 200, according to a first configuration.

The sub-probe 208 comprises an optical fiber end 210 coated, around its circumference, with an electrode 212 of electrically conductive material, using methods and materials known per se.

The optical fiber at the optical fiber end 210 may be a quartz fiber.

As shown, the optical fiber end 210 may be split in two parts, forming a bundle of a first sub-fiber end 210a and a second sub-fiber end 210b.

The first sub-fiber end 210a and the second sub-fiber end 210b may be glued together, using methods known per se. For example, as shown, the fibers may have been polished into having a half-circular cross section before being glued together.

As the probe 200 (cf. Fig. 1) is pushed against a section of skin 202 (cf. Fig. 1), both the fiber ends 210 and the electrodes 212 are brought into contact with the skin 202. Light may be shone by, for example the first sub-fiber end 210a on the skin 202 (cf. Fig. 1), as indicated by an outward-pointing arrow in Fig. 3a, scattered by the skin 202 and picked up by the second sub-fiber end 210b, as indicated by an inward-pointing arrow in Fig. 3a.

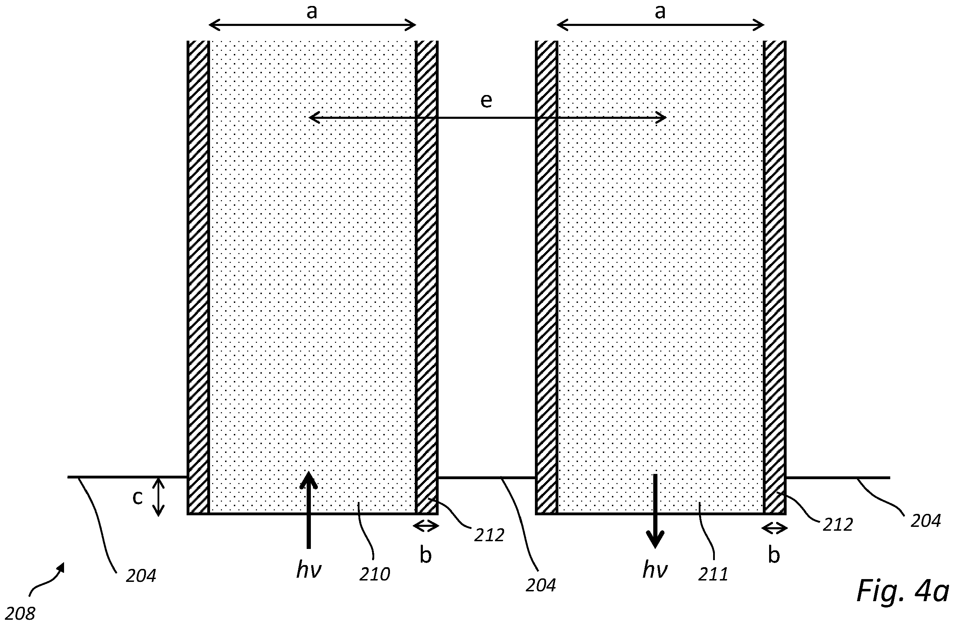

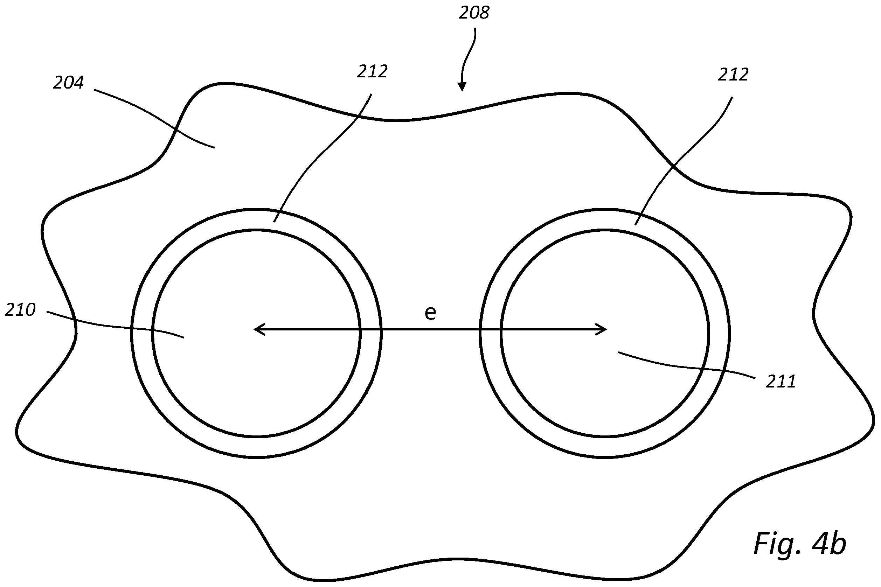

Figs 4a and 4b show, respectively, in a cross-sectional view (Fig. 4a) and a front view (Fig. 4b), a skin-facing sub-probe 208 of the array 206 of skin-facing sub-probes 208 (cf. Fig. 2) as located at the front surface 204 of the probe 200, according to a second configuration. The sub-probe 208 comprises a first optical fiber end 210 and a second optical fiber end 211, each coated, around its circumference, with an electrode 212 of electrically conductive material, using methods and materials known per se. Alternatively (not shown) only one of the first optical fiber-end 210 and the second optical fiber end 211 may be coated with such an electrode 212.

In this case, as the probe 200 (cf. Fig. 1) is pushed against a section of skin 202 (cf. Fig. 1), both the two fiber ends 210, 212, and the electrodes 212, are brought into contact with the skin 202. Light may be shone by, for example, as indicated by an outward-pointing arrow in Fig. 4a, the first fiber end 210 on the skin 202 (cf. Fig. 1), scattered by the skin 202 and picked up by the second fiber end 211, as indicated by an inward-pointing arrow in Fig. 4a.

In both the first configuration of Figs 3a and 3b and the second configuration of Figs 4a and 4b, the optical fiber ending at the optical fiber end 210 may be a quartz fiber.

In both the first configuration (cf. Figs 3a and 3b) and the second configuration (cf. Figs 4a and 4b), electrodes 212 may be made from any electrically-conductive material, for example from metal, from conducting carbon, or from conducting polymer.

In both the first configuration (cf. Figs 3a and 3b) and the second configuration (cf. Figs 4a and 4b), a fiber end 210 may have a diameter, i.e., a maximum transversal extent, a, in the interval, 0.25 mm - 4.0 mm, preferably 0.5 mm - 2.0 mm.

In both the first configuration (cf. Figs 3a and 3b) and the second configuration (cf. Figs 4a and 4b), an electrode 212 may have a maximum transversal thickness, b, in the interval, 0.03 mm - 0.5 mm, preferably 0.06 mm - 0.25 mm.

In both the first configuration (cf. Figs 3a and 3b) and the second configuration (cf. Figs 4a and 4b), the sub-probe 208 may be raised a distance *c* from the front surface 204, or lie flat with the front surface 204, i.e., the distance c being zero. The distance c may lie in the interval 1 mm - 5 mm.

In the second configuration (cf. Figs 4a and 4b), the center spacing (cf. Fig. 4a) between the first optical fiber end 210 and the second optical fiber end 211 e may for example be equal to or less than the diameter a+2b of a fiber end 210, 211 with coating 212 or equal to or less than the diameter a of a fiber end 210, 211. This may assure optical coupling between the first fiber end 210 and the second fiber end 211 through scattering on the skin 202.

Referring back to Fig. 1, the cable 104 carries optical fibers from each respective fiber end 210 of the sub-probes 208 and electric leads from each respective electrode 212 of the sub-probes 208.

As shown, at the main unit 102, the cable 104 may be split into an optical bundle 106, carrying the optical fibers, and an electric bundle 108, carrying the electric leads.

The optical cable 106 may lead to an optical switch device 110, comprising a (schematically drawn) array of fiber switches 111, as known per se, configured to switchably connect a fiber of the optical cable 106, connected to a respective fiber end 210 (cf. Figs. 3a, 3b, 4a, 4b) of the plurality of optical fiber ends 210 to a light measurement device 112, which may have an input 128 and an output 126.

For light absorbance measurement at a sub-probe 208 according to the first configuration (cf. Figs 3a and 3b), the optical switch device may be switched so that light is output 126 at the first sub-fiber end 210a and input 128 from the second sub fiber end 210b.

Similarly, for light absorbance measurement at a sub-probe 208 according to the second configuration (cf. Figs 4a and 4b), the optical switch device may be switched so that light is output at the first fiber end 210 and input from the second fiber end 211.

The light measurement device 112 may thus, through the switching of the array of fiber switches 111 of the optical switch device 110, be configured for the light absorbance measurement through a plurality of optical fiber ends 210 of the skin-facing sub-probes 208.

For each optical fiber end 210, as switched to the light measurement device 112 by the array of optical switches 111 of the light switch device 110, the light measurement device may be configured for near infra-red, NIR, light absorbance measurement.

For each optical fiber end, as switched to the light measurement device 112 by array of optical switches 111 of the light switch device 110, the light measurement device may be configured for light absorbance measurement at a plurality of wavelengths, which may include or be exclusive to NIR wavelengths.

The electric bundle 108 may lead to an electric switch device 114, comprising a schematically-drawn plurality of microswitches 116 configured to switchably connect pairs of electrodes 212 (cf. Fig. 2b) of the plurality of electrodes located at the sub-probes 208 (Fig. 2b) to an impedance measurement device 118.

The impedance measurement device 118 has a first input 120 and a second input 122, between which the impedance measurement device 118 may measure the electric impedance. Thereby, through the array of microswitches 116 in the electric switch device 114, the impedance measurement device 118 may be configured for pairwise impedance measurement across a plurality of pairs of electrodes 212 of the skin-facing sub-probes 208, in particular between different sub-probes 208. Typically, pairwise measurements between all electrodes 212 will be possible through switching in the plurality of microswitches 116.

For each pair of electrodes 212 as switched to the impedance measurement device 118 through the electric switching device 114, impedance measurement may be performed at a plurality of frequencies. The plurality of frequencies may, for example, lie in the interval 100 Hz - 10 MHz. The impedance may, for example, be calculated from combined measurement of voltage and current.

The system 100 may further comprise a processor 124 configured to interpolate and/or extrapolate a map of skin properties based on the combined impedance and light measurements of the light measurement device 112 and the impedance measurement device 118. The processor 124 may, as shown be integrated in the main unit 124, or be separate.

As an alternative, the optical switch device 110 and the electric switch may be comprised in the probe 200 rather than the main unit 102. Further, again as an alternative, one or more of the optical switch device 110, the optical measurement device 112, the electric switch device 114, the impedance measurement device 118, and processor 124 may be separate from the main unit.

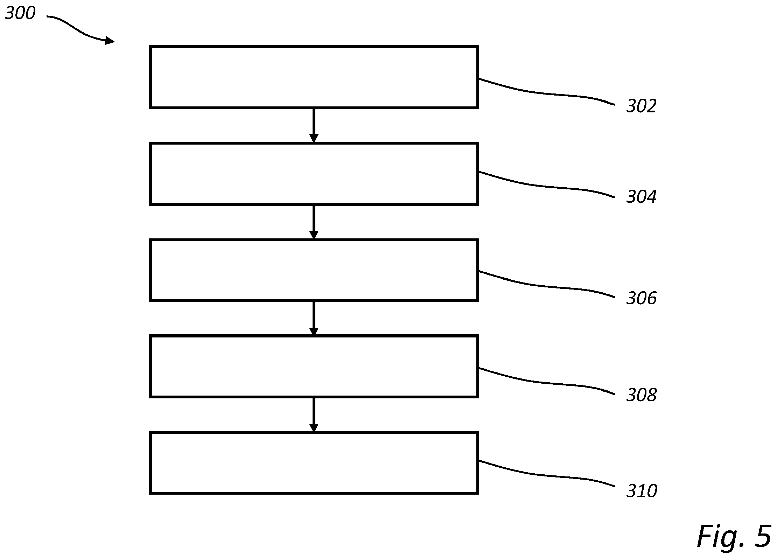

In the following, a typical measurement sequence 300 will be described with reference to Fig. 5.

At 302, the probe is placed on a skin 202 surface of interest.

At 304, the optical switch device 110 may, in sequence, switch each of the optical fibers leading to skin-facing optical fiber ends 210 at each sub-probe 208 to the light measurement device 112, which may perform light measurement as described above on one or several wavelengths.

At 306, the electric switch 114, may, in sequence, switch the inputs 120 and 122 of the impedance measurement device 118 to pairwise combinations of sub-probe electrodes 212 and perform impedance measurement as described above at one or several frequencies.

Steps 302 and 304 may either be performed in sequence one after the other, or fully or partially overlapping. No matter whether steps 302 and 304 are performed in sequence or overlapping, simultaneous impedance and light measurement is achieved, as the probe 200 does not need to be operated or moved between light and impedance measurements.

At 308, measurement results from the light measurement device 112 and the impedance measurement device 118 may be transferred to the processor 120 for further analysis.

At 310, the processor interpolates and/or extrapolates a map of skin properties based on the combined impedance and light measurements. The signals from the plurality of sub-probes may, through mathematical interpolation allow the reconstruction of intensity contour levels of a parameter measured or calculated from multivariate data, showing how skin properties change over the surface, i.e., allowing construction of a map over the skin area covered by the probe.

The inventive concept has mainly been described above with reference to example embodiments. However, as is readily appreciated by a person skilled in the art, other embodiments than the ones disclosed above are equally possible within the scope of the inventive concept, as defined by the appended patent claims.

## Claims

1. A probe (200) for combined impedance and light absorbance measurement on skin (202), comprising a two-dimensional array (206) of skin-facing sub-probes (208), each said skin-facing sub-probe (208) comprising an optical fiber end (210, 211) coated, around its circumference, with an electrode (212).

2. The probe of claim 1, wherein said electrode (212) is made from metal, conducting carbon, or conducting polymer.

3. The probe of any one of claims 1-2, wherein a said fiber is a quartz fiber.

4. The probe of any one of claims 1-3, wherein the sub-probes (208) of said two-dimensional array (206) are arranged in a square, rectangular, circular, elliptic, or hexagonal pattern.

5. The probe of any one of claims 1-4, wherein a said fiber end (210) has a maximum transversal extent in the interval, 0.25 mm - 4.0 mm, preferably 0.5 mm - 2.0 mm.

6. The probe of any one of claims 1-5, wherein a said electrode (212) has a maximum transversal thickness in the interval, 0.03 mm - 0.5 mm, preferably 0.06 mm - 0.25 mm.

7. A measurement system (100) configured for simultaneous impedance and light measurement on skin (202), comprising:
the probe (200) of any one of claims 1-6;
an impedance measurement device (118) configured for pairwise said impedance measurement across a plurality of pairs of electrodes (212) of said skin-facing sub-probes (208); and
a light measurement device (112) configured for said light absorbance measurement through a plurality of optical fiber ends (210, 211) of said skin-facing sub-probes.

8. The system of claim 7, further comprising a plurality of microswitches (116) configured to switchably connect pairs of electrodes (212) of said plurality of electrodes to said impedance measurement device (118) for said pairwise impedance measurement.

9. The system of any one of claims 7-8, configured for said impedance measurement at a plurality of frequencies.

10. The system of any one of claims 7-9, further comprising an array of optical switches (111) configured to switchably connect a fiber connected to a fiber end (110, 111) of said plurality of optical fiber ends to said light measurement device (112) for said light absorbance measurement.

11. The system of any one of claims 7-10, wherein said light measurement device (112) is configured for near infra-red, NIR, said light absorbance measurement.

12. The system of any one of claims 7-11, wherein said light measurement device (112) is configured for said light absorbance measurement at a plurality of wavelengths.

13. The system of any one of claims 7-12, further comprising a processor (124) configured to interpolate and/or extrapolate a map of skin (202) properties based on said combined impedance and light measurement.

14. Use of the probe (200) of any one of claims 1-6 or the system (100) of any one of claims 7-13 in combined impedance and light absorbance measurement on skin (202).
